# EUROPÄISCHE PATENTSCHRIFT

(11) **EP 3 017 296 B1**
(45) Veröffentlichungstag und Bekanntmachung des Hinweises auf die Patenterteilung: **23.02.2022**
(21) Anmeldenummer: 14736349.3
(22) Anmeldetag: 01.07.2014
(51) Int. Cl.: G01N 27/447, G01N 1/28, G02B 21/32, B01L 3/00

(54) **LASERMIKRODISSEKTIONSSYSTEM UND UNTERSUCHUNGSVERFAHREN FÜR NUKLEINSÄUREHALTIGE PROBEN**
LASER MICRODISSECTION SYSTEM AND EXAMINATION METHOD FOR SAMPLES CONTAINING NUCLEIC ACID
SYSTÈME DE MICRODISSECTION LASER ET PROCÉDÉ D'ANALYSE D'ÉCHANTILLONS CONTENANT DE L'ACIDE NUCLÉIQUE

(30) Priorität: 01.07.2013 DE 102013212811
(43) Veröffentlichungstag der Anmeldung: 11.05.2016
(73) Patentinhaber: Leica Microsystems CMS GmbH, 35578 Wetzlar (DE)
(72) Erfinder: SCHLAUDRAFF, Falk, 35510 Butzbach (DE)
(74) Vertreter: Bradl, Joachim
(86) Internationale Anmeldenummer: PCT/EP2014/063920
(87) Internationale Veröffentlichungsnummer: WO 2015/000879

(56) Entgegenhaltungen:
- DE-A1- 10 018 251
- DE-A1- 10 057 292
- DE-A1-102009 016 512
- US-A- 5 671 086
- US-A1- 2004 247 777
- US-A1- 2008 069 737
- US-A1- 2009 272 657
- US-A1- 2012 058 467

## Beschreibung

Die vorliegende Erfindung betrifft ein Lasermikrodissektionssystem sowie ein entsprechendes Untersuchungsverfahren für nukleinsäurehaltige Proben gemäß unabhängigen Patentansprüche und

### Stand der Technik

Verfahren zur Bearbeitung biologischer Proben durch die sogenannte Lasermikrodissektion existieren bereits seit Mitte der 1970er Jahre (siehe z.B. Isenberg, G. et al.: Cell surgery by laser micro-dissection: a preparative method. Journal of Microscopy, Band 107, 1976, Seiten 19-24) und wurden seitdem kontinuierlich weiterentwickelt.

Bei der Lasermikrodissektion können Zellen, Geweberegionen usw. aus einer Probe ("Objekt", "Präparat") isoliert und als sogenannte Dissektate gewonnen werden. Ein besonderer Vorteil der Lasermikrodissektion ist der kurze Kontakt der Probe mit dem Laserstrahl, durch den die Probe kaum verändert wird. Die spezifische Gewinnung der Dissektate kann dabei auf unterschiedliche Weise erfolgen (siehe z.B. Bancroft, J.D. und Gamble, M.: Theory and Practice of Histological Techniques. Elsevier Science, 2008, Seite 575, Kapitel "Laser Microdissection").

Beispielsweise kann in bekannten Verfahren aus einer Probe mittels eines Infrarot- oder Ultraviolettlaserstrahls ein Dissektat isoliert werden, das unter dem Einfluss der Schwerkraft in einen geeigneten Dissektatauffangbehälter fällt. Das Dissektat kann dabei aus der Probe auch zusammen mit einer anhaftenden Membran ausgeschnitten werden. Bei der sogenannten Laser Capture Microdissection wird hingegen eine thermoplastische Membran mittels eines entsprechenden Laserstrahls erwärmt. Dabei verschmilzt die Membran mit dem gewünschten Bereich der Probe und kann in einem darauffolgenden Schritt durch Reißen entfernt werden. Eine weitere Alternative besteht darin, das Dissektat mittels des Laserstrahls an einen Deckel eines Dissektatauffangbehälters anzuheften. Bei bekannten inversen Mikroskopsystemen zur Lasermikrodissektion können nach oben katapultierte Dissektate auch an den Boden eines Dissektatauffangbehälters, der mit einer adhäsiven Beschichtung versehen ist, angeheftet werden.

Die vorliegende Erfindung kommt hierbei insbesondere bei den Verfahren zum Einsatz, bei denen ein Dissektat aus einer Probe herausgetrennt und in einem darunter angeordneten Dissektatauffangbehälter aufgefangen wird. Insbesondere ist die Erfindung für kontaktfreie Auffangsysteme für Dissektate geeignet.

Bekannte Mikroskopsysteme zur Lasermikrodissektion, wie sie beispielsweise aus der WO 98/14816 A1 bekannt sind, weisen eine Auflichteinrichtung auf, in deren Strahlengang ein Laserstrahl eingekoppelt wird. Der Laserstrahl wird durch das jeweils verwendete Mikroskopobjektiv auf die Probe fokussiert, die auf einem motorisch-automatisch verfahrbaren Mikroskoptisch aufliegt. Eine Schnittlinie wird dadurch erzeugt, dass der Mikroskoptisch beim Schneiden verfahren wird, um die Probe relativ zu dem feststehenden Laserstrahl zu bewegen. Dies hat jedoch unter anderem den Nachteil, dass die Probe während des Erzeugens der Schnittlinie nicht ohne weiteres betrachtet werden kann, da diese sich im Gesichtsfeld bewegt und das Bild verschwommen erscheint.

Vorteilhafter sind daher Lasermikrodissektionssysteme, die Laserablenk- bzw. Laserscaneinrichtungen aufweisen, die dazu eingerichtet sind, den Laserstrahl bzw. dessen Auftreffpunkt über die fest stehenden Probe zu lenken. Derartige Lasermikrodissektionssysteme, die auch im Rahmen der vorliegenden Erfindung besondere Vorteile bieten, werden unten im Detail erläutert. Ein besonders vorteilhaftes Lasermikrodissektionssystem, das eine Laserscaneinrichtung mit gegeneinander verstellbaren Glaskeilen im Laserstrahlengang aufweist, ist beispielsweise in der bereits zuvor erwähnten EP 1 276 586 B1 beschrieben.

In beiden Fällen, also sowohl in Lasermikrodissektionssystemen, in denen der Mikroskoptisch verfahren wird, als auch in Lasermikrodissektionssystemen, die eine Laserscaneinrichtung aufweisen, wird in der Regel mit gepulsten Lasern gearbeitet, wobei durch jeden Laserpuls ein Loch in der Probe erzeugt wird. Eine Schnittlinie entsteht durch eine Aneinanderreihung derartiger Löcher, gegebenenfalls mit einer entsprechenden Überlappung.

Die Lasermikrodissektion kann zur Gewinnung von Einzelzellen oder definierten Gewebebereichen, also Dissektaten nukleinsäurehaltiger Proben, verwendet werden. Entsprechende Dissektate können anschließend unterschiedlichen molekularbiologischen Analyseverfahren unterworfen werden.

Zur Untersuchung von DNA-Schädigungen (insbesondere von Einzel- oder Doppelstrangbrüchen, Multiplikationen, Deletionen, Dimerisierungen usw.) in Einzelzellen oder bestimmten Gewebebereichen ist beispielsweise die Einzelzell-Gelelektrophorese bekannt (siehe z.B. Wood, D.K. et al.: Single cell trapping and DNA damage analysis using microwell arrays, Proc. Natl. Acad. Sci. USA, Band 107, 2010, Seiten 10.008-10.013). Die Einzelzell-Gelelektrophorese (auch als "Comet-Assay" bezeichnet) beruht darauf, dass entsprechende DNA-Schädigungen, beispielsweise Strangbrüche in der DNA, Abweichungen in deren geometrischen Eigenschaften und damit im Migrationsverhalten bzw. der Mobilität in der Elektrophorese verursachen.

Im Allgemeinen kann hierbei beobachtet werden, dass geschädigte DNA-Fragmente in den üblicherweise verwendeten Agarosegelen mobiler sind als ungeschädigte DNA-Fragmente. Dies äußert sich in einem kometenschweifähnlichen Migrationsbild. Durch die Einzelzell-Gelelektrophorese kann eine Vielzahl unterschiedlicher DNA-Läsionen erkannt werden, wobei beispielsweise auch DNA-Reparaturenzyme oder andere Reagenzien zum Einsatz kommen können, um an sich nicht detektierbare Schädigungen sichtbar zu machen. Für eine Übersicht sei auf den zuvor erwähnten Artikel von Wood et al. verwiesen.

Die Einzelzell-Gelelektrophorese kann jedoch herkömmlicherweise nur mit geringem Durchsatz und verhältnismäßig schlechter Reproduzierbarkeit durchgeführt werden. Die verwendeten Bildverarbeitungs- und -analyseverfahren sind aufwändig, zeitraubend und potentiell mit Fehlern behaftet. Der Artikel von Wood et al. schlägt zwar ein Verfahren zur Hochdurchsatz-Einzelzell-Gelelektrophorese vor, wobei ein Elektrophoresegel mit einer Anzahl von Geltaschen verwendet wird, das anschließend Standard-Hochdurchsatz-Screeningtechniken unterworfen werden kann. Insbesondere die gezielte Einbringung bestimmter Zellen oder Zelltypen in bestimmte Geltaschen ist jedoch mittels des dort offenbarten Verfahrens nicht möglich. Das gesamte Gel wird dort mit einer Zellsuspension überschichtet und die jeweiligen Zellen sedimentieren zufällig in die jeweils vorhandenen Geltaschen.

Die Erfindung will daher die Durchführung von entsprechenden Einzelzell-Gelelektrophoreseverfahren verbessern.

DE 100 18 251 A1 offenbart ein Laser-Mikrodissektionssystem mit einem Mikroskop und Positionierungsmitteln zum Positionieren einer Auffangvorrichtung parallel zur Objektebene und relativ zu einer definierten Bezugsposition (optische Achse) umfasst, so dass mittels des Laserstrahls gewonnene Dissektate einer in der Objektebene angeordneten Probe in mindestens einem Behälter gesammelt werden können, wobei die Positionierungsmittel geeignet sind, den Behälter relativ zur Probe zu positionieren.

### Offenbarung der Erfindung

Vor diesem Hintergrund schlägt die vorliegende Erfindung ein Lasermikrodissektionssystem, eine Elektrophoreseeinheit für ein derartiges Lasermikrodissektionssystem, sowie ein entsprechendes Untersuchungsverfahren für nukleinsäurehaltige Proben mit den Merkmalen der unabhängigen

Patentansprüche 1 und 10 vor. Bevorzugte Ausgestaltungen sind jeweils Gegenstand der abhängigen Patentansprüche sowie der nachfolgenden Beschreibung.

### Vorteile der Erfindung

Die Erfindung geht von einem an sich bekannten Lasermikrodissektionssystem aus. Ein derartiges Lasermikrodissektionssystem umfasst ein Mikroskop, das eine Auflichteinrichtung, ein Mikroskopobjektiv und eine Lasereinheit aufweist, wobei ein Strahlengang eines Laserstrahls der Lasereinheit durch die Auflichteinrichtung und durch das Mikroskopobjektiv verläuft und eine Objektebene des Mikroskopobjektivs an einem einstellbaren Schnittpunkt schneidet. Erfindungsgemäß zeichnet sich das Lasermikrodissektionssystem dadurch aus, dass unterhalb der Objektebene eine Elektrophoreseeinheit mit einem Elektrophoresegel angebracht ist, wobei das Elektrophoresegel zumindest eine Geltasche aufweist und das Lasermikrodissektionssystem Positionierungsmittel zum Positionieren des Elektrophoresegels parallel zu der Objektebene und relativ zu einer definierten Bezugsposition umfasst, so dass mittels des Laserstrahls der Lasereinheit gewonnene Dissektate einer in der Objektebene anordenbaren Probe in der zumindest einen Geltasche auffangbar sind.

Ein derartiges Lasermikrodissektionssystem umfasst, mit anderen Worten, ein Mikroskop mit einer Auflichteinrichtung zum Fokussieren eines Laserstrahls durch ein Mikroskopobjektiv des Mikroskops auf eine zu dissektierende, insbesondere nukleinsäurehaltige Probe, und Mittel zum Heraustrennen eines Dissektats aus der nukleinsäurehaltigen Probe mittels des fokussierten Laserstrahls.

Die Erfindung kommt dabei insbesondere in sogenannten kontaktfreien Lasermikrodissektionssystemen zum Einsatz. Diese zeichnen sich dadurch aus, dass ein Dissektat nicht an Membranen und dergleichen angeheftet wird, sondern aufgrund der Schwerkraft aus der Probe nach unten fällt. Die Probe ist dabei in Aufrechtsystemen an der Unterseite eines Objektträgers angeordnet und wird durch den Objektträger hindurch von dessen Oberseite aus mit dem Laserstrahl bearbeitet.

Mittels der Auflichteinrichtung wird ein Laserstrahl aus einer Laserlichtquelle in den Beobachtungsstrahlengang des Mikroskops eingekoppelt. Der Laserstrahl wird durch das Mikroskopobjektiv, das auch zum Betrachten der nukleinsäurehaltigen Probe verwendet wird, auf die Probe fokussiert.

Zur Vermeidung von Missverständnissen sei an dieser Stelle betont, dass das im Rahmen der Erfindung eingesetzte Lasermikrodissektionssystem mit nukleinsäurehaltigen Proben verwendet wird, die bereits mikroskopietauglich vorbereitet sind. Hierbei kann es sich beispielsweise um Dünnschnitte handeln, die mittels eines Mikrotoms aus einem größeren Gewebeblock herausgetrennt werden. Bei einem solchen Gewebeblock kann es sich beispielsweise um ein fixiertes Organ oder eine Biopsie eines entsprechenden Organs handeln. Das erfindungsgemäße Lasermikrodissektionssystem dient daher nicht zur Gewinnung von nukleinsäurehaltigen Proben sondern zu deren Bearbeitung sowie zur Isolation von bestimmten Bereichen hiervon. Es versteht sich, dass die Erfindung auch mit anderen nukleinsäurehaltigen Proben, die nicht mittels eines Mikrotoms gewonnen werden, zum Einsatz kommen kann, beispielsweise mit Ausstrichen, Mazeraten usw.

Mikrotome werden ausschließlich bei der Vorbereitung von mikroskopischen Proben eingesetzt. Mikrotome können hierzu auch Laser aufweisen. Die mittels eines Mikrotoms erhaltenen Schnitte werden auf einen Objektträger, wie oben erwähnt, aufgebracht, ggf. dort befestigt, angefärbt usw. Erst dann stehen diese für einen Einsatz in dem erfindungsgemäßen Lasermikrodissektionssystem zur Verfügung. Ein Mikrotom unterscheidet sich in seinem Betrieb unter anderem dadurch fundamental von einem Lasermikrodissektionssystem, dass dort Schnitte mit möglichst homogener Schnittstärke gewonnen werden. Mikrotome sind daher dazu ausgebildet, eine große Anzahl an identischen Schnitten mit parallelen Schnittflächen zu erzeugen, wohingegen Lasermikrodissektionssysteme zum Heraustrennen von Dissektaten nach probenabhängigen Kriterien, beispielsweise nach visuellen Kriterien, eingerichtet sind. Der Fachmann würde daher bei Mikrotomen eingesetzte technische Lösungen nicht auf Lasermikrodissektionssysteme übertragen.

Mikrotome umfassen ferner keine Mikroskope, in deren Beobachtungsstrahlengang ein Laserstrahl eingekoppelt wird. Der Laserstrahl wird daher in Mikrotomen auch niemals durch ein Mikroskopobjektiv, das auch zur Betrachtung verwendet wird, auf eine bearbeitete Probe, z.B. einen Gewebeblock, fokussiert.

Ferner kommt im Rahmen der vorliegenden Erfindung vorteilhafterweise ein Lasermikrodissektionssystem mit einer Laserscaneinrichtung zum Einsatz. Der Mikroskoptisch ist in Lasermikrodissektionssystemen mit einer Laserscaneinrichtung bezüglich der x-y-Richtung (also in den Richtungen senkrecht zur optischen Achse des Mikroskopobjektivs) beim Heraustrennen des Dissektats, also während des Dissektiervorgangs, feststehend angeordnet.

Im Gegensatz zu Lasermikrodissektionssystemen mit einem während des Dissektiervorgangs motorisch verfahrenen Mikroskoptisch (Scanningtisch), der insbesondere bei stark vergrößernden Objektiven eine hohe Positioniergenauigkeit besitzen muss, um präzise Schnitte zu ermöglichen, erweisen sich Lasermikrodissektionssysteme mit einer Laserscaneinrichtung als einfacher und kostengünstiger in der Herstellung und besitzen Präzisionsvorteile.

Die Laserscaneinrichtung weist in einer besonders vorteilhaften Ausführungsform zwei dicke, gegen eine optische Achse geneigte und unabhängig voneinander um die optische Achse drehbare gläserne Keilplatten auf, welche durch ihre Keilwinkel eine Strahlablenkung erzeugen. Durch die Drehung der gläsernen Keilplatten ist der resultierende Ablenkwinkel des Laserstrahls gegenüber der optischen Achse variabel. Am Ausgang der Laserscaneinrichtung weist der Laserstrahl durch die Dicke und die Schrägstellung der gläsernen Keilplatten einen seitlichen Strahlversatz gegenüber der optischen Achse auf und trifft für alle Ablenkwinkel die Mitte der Objektivpupille des Mikroskopobjektivs. Der Schnittpunkt des Laserstrahls mit der Objektebene ist damit einstellbar.

Eine derartige Laserscaneinrichtung ist insbesondere deshalb vorteilhaft gegenüber anderen Laserscaneinrichtungen wie beispielsweise Spiegelscannern, Galvanometerscannern oder Schrittmotorscannern, weil diese nicht in einer zu der Objektivpupille konjugierten Ebene angeordnet werden muss. Damit ist auch keine sogenannte Pupillenabbildung erforderlich, um zu erreichen, dass der abgelenkte Strahl die Objektivpupille trifft. Bei der Mikrodissektion mit UV-Laserlicht wäre dabei beispielsweise eine UV-taugliche Pupillenabbildung erforderlich. Weitere Vorteile einer derartigen Laserscaneinrichtung mit Keilplatten sind beispielsweise in der EP 1 276 586 B1 genannt.

Unter einer Elektrophoreseeinheit, die "angebracht" ist, sei verstanden, dass die Elektrophoreseeinheit dauerhaft in einem entsprechenden Lasermikrodissektionssystem befestigt ist oder Kopplungsmittel aufweist, um diese vorübergehend in einem entsprechenden Lasermikrodissektionssystem zu befestigen. Eine "dauerhafte" Befestigung kann beispielsweise mittels einer Verschraubung erfolgen, schließt jedoch nicht aus, dass eine derartige Befestigung beispielsweise zur Wartung oder bei einer Umkonfiguration eines entsprechenden Lasermikrodissektionssystems gelöst werden kann.

Für eine vorübergehende Befestigung vorgesehene Kopplungsmittel umfassen beispielsweise seitens des Mikroskops des Lasermikrodissektionssystems ausgebildete Anbringungsstrukturen. Auch die Elektrophoreseeinheit weist entsprechende (komplementäre) Anbringungsstrukturen auf. Beispielsweise kann ein Lasermikrodissektionssystem geeignete Schienen oder Aussparungen aufweisen, die zum Einschieben bzw. Einsetzen einer Elektrophoreseeinheit eingerichtet sind. Die Elektrophoreseeinheit weist in diesem Fall beispielsweise komplementäre Schienen und/oder eine Form auf, die ein Einsetzen in einer entsprechenden Aussparung ermöglichen. Solche Schienen, Formen und/oder Aussparungen können (sowohl seitens der Elektrophoreseeinheit als auch z.B. seitens des Mikroskops des Lasermikrodissektionssystems) derart ausgebildet sein, dass sie die Anbringung einer Elektrophoreseeinheit nur in einer vorgegebenen Position ermöglichen, beispielsweise um Benutzerfehler zu vermeiden, oder um eine reproduzierbare Positionierung relativ der definierten Bezugsposition in der Objektebene zu erzielen.

Bei der "definierten Bezugsposition" kann es sich um jeden Fixpunkt handeln, bezüglich dessen ein Objekt fixiert oder fixierbar ist, und das eine Bezugnahme auf Regionen in dem Objekt ermöglicht. Eine definierte Bezugsposition braucht dabei nicht selbst in der Objektebene festgelegt werden, es reicht aus, dass diese Bezugsposition ihrerseits eine definierte Lage gegenüber einem weiteren Bezugspunkt in dem verwendeten Lasermikrodissektionssystem aufweist. Beispielsweise kann eine definierten Bezugsposition ein Schnittpunkt der Objektivachse mit der Objektebene sein. Durch das Positionieren des Elektrophoresegels parallel zu der Objektebene und relativ zu der definierten Bezugsposition kann jeweils eine Geltasche als Auffangeinrichtung für ein Dissektat bereitgestellt werden, so dass mittels des Laserstrahls der Lasereinheit gewonnene Dissektate einer in der Objektebene anordenbaren Probe in der zumindest einen Geltasche auffangbar sind.

Ein "Elektrophoresegel" ist in gängiger Weise hergestellt und kann beispielsweise aus ein- oder zweiprozentiger Agarose mit definiertem Schmelzpunkt, ggf. mit geeigneten Zusätzen, bestehen. Es weist mindestens eine, vorzugsweise jedoch mehrere, Geltaschen auf. Details zu dem Elektrophoresegel und dessen Geltaschen werden unten erläutert.

Das Lasermikrodissektionssystem weist also Mittel zur Positionierung des Elektrophoresegels mit der oder den Geltaschen relativ zu der nukleinsäurehaltigen Probe auf. In der oder den Geltaschen können hierdurch die Dissektate aufgefangen werden. Typischerweise umfasst dabei eine "Positionierung" eine Anordnung einer Geltasche unterhalb der Probe bzw. deren zu dissektierendem Bereich.

Mittels des erfindungsgemäßen Lasermikrodissektionssystems können im Gegensatz zum Stand der Technik gezielt Einzelzellen oder Geweberegionen (Zellverbände) einer nukleinsäurehaltigen Probe in entsprechende Geltaschen überführt und gezielt untersucht werden. Die Einzelzellen oder Geweberegionen können nach der Durchführung eines entsprechenden Elektrophoreseverfahrens, beispielsweise des erläuterten Comet-Assays, eindeutig bestimmten Bereichen der Probe zugeordnet werden. Es wird also jeweils eine Zuordnung genau eines Signals eines entsprechenden Elektrophoreseverfahrens zu einem definierten Probenbereich möglich. Im Gegensatz zum Stand der Technik, bei dem eine gezielte Isolierung von Einzelzellen bzw. Probenbereichen wie Geweben oder Zellverbänden nicht möglich ist, erlaubt die vorliegende Erfindung beispielsweise gezielte und lokal aufgelöste Aussagen über eine DNA-Schädigung in derartigen Einzelzellen bzw. Probenbereichen.

Bei der Probe kann es sich beispielsweise um Schnitte von festen Geweben, Aus- oder Abstriche, beispielsweise von medizinischen Proben, adhärente Zellkulturen und dergleichen handeln. Ein besonderer Vorteil der Erfindung ist der, dass unterschiedliche Bereiche derartiger Proben unterschiedlich behandelt werden können (z.B. mittels ionisierender Strahlung und/oder mutagenen Reagenzien) und eine entsprechende Reaktion (in Form einer DNA-Schädigung) lokal erfassbar ist. Weil die Proben im Übrigen gleich behandelt werden können, werden aus der Probenbearbeitung resultierende Unterschiede weitgehend vermieden.

Die Erfindung ermöglicht auch die Erfassung zell- oder gewebespezifischer Reaktionen auf entsprechende schädigende Faktoren, indem beispielsweise entsprechende Bereiche eines mikroskopischen Schnitts untersucht werden und mit nicht affektierten (d.h. beispielsweise nicht mittels ionisierender Strahlung und/oder mutagenen Reagenzien behandelten) Zell- oder Gewebeproben verglichen werden. Dies erweist sich als bedeutend einfacher als die VorabIsolierung mittels alternativer Verfahren und die klassische Durchführung einer Einzelzell-Gelelektrophorese. Wie erwähnt, muss hierzu bisher eine Zellsuspension hergestellt werden, aus der die entsprechenden Zellen oder Gewebebereiche in die Geltaschen sedimentieren.

Eine "Elektrophoreseeinheit" kann im Rahmen der vorliegenden Erfindung je nach Integrationsgrad der Elektrophorese in das Lasermikrodissektionssystem unterschiedlich ausgebildet sein und insbesondere eine unterschiedliche Ausstattung mit zusätzlichen Komponenten aufweisen. Wie nachfolgend erläutert, kann die Elektrophoreseeinheit zur Durchführung der eigentlichen Elektrophorese in dem Lasermikrodissektionssystem verbleiben oder diesem entnommen werden. Im letzteren Fall kann die Elektrophoreseeinheit auch ausgesprochen einfach ausgebildet sein und beispielsweise nur das Elektrophoresegel auf einem geeigneten Gelträger umfassen. Vorteilhafterweise sind hierbei Mittel vorgesehen, die eine definierte Positionierung oder Ausrichtung des Elektrophoresegels in dem Lasermikrodissektionssystem und damit eine Zuordnung ermöglichen und die Positionierung speicherbar machen. So kann beispielsweise ein Gelträger geeignete Markierungen zu dessen Ausrichtung in dem Lasermikrodissektionssystem umfassen, wobei die Position jeder einzelnen Geltasche beispielsweise unter Bezugnahme auf ein durch den Gelträger oder das Lasermikrodissektionssystem festgelegtes Koordinatensystem - die definierte Bezugsposition - definiert ist.

Die Elektrophoreseeinheit kann fest an diese angebrachte oder mittels Kopplungsmitteln anbringbare Fluidleitungen aufweisen, die an entsprechende Mittel zum Einbringen von Fluid in die Elektrophoreseeinheit und/oder zum Entnehmen von Fluid aus dieser angeschlossen sind. Zur Bedeutung der Formulierung "angebracht oder mittels Kopplungsmitteln anbringbar" sei auf die obigen Erläuterungen verwiesen. Beispielsweise können entsprechende Fluidleitungen dazu verwendet werden, die Elektrophoreseeinheit vor der Durchführung der Elektrophorese mit einem geeigneten Gelmaterial, beispielsweise Agarose, zu überschichten und/oder einen Elektrophoresepuffer und/oder geeignete Nachweisreagenzien einzubringen. Als Elektrophoresepuffer und/oder Nachweisreagenzien können bekannte Gemische bzw. Verbindungen verwendet werden. Mittels entsprechender Fluidleitungen kann Fluid selbstverständlich auch aus der Elektrophoreseeinheit entnommen werden. Es können auch gasförmige Fluide verwendet werden, beispielsweise um eine (geschlossene) Elektrophoreseeinheit zu trocknen und/oder andere Fluide zu verdrängen.

Vorteilhafterweise kann eine entsprechende Elektrophoreseeinheit auch bereits über geeignete Elektroden zum Anlegen einer Spannung an das Elektrophoresegel verfügen. Derartige Elektroden erstrecken sich vorteilhafterweise entlang zweier gegenüberliegender Seiten des Elektrophoresegels.

Wie erwähnt, kann ein erfindungsgemäßes Lasermikrodissektionssystem dazu eingerichtet sein, eine Elektrophorese mit der in dem Lasermikrodissektionssystem angebrachten Elektrophoreseeinheit durchzuführen. Hierzu kann ein entsprechendes Lasermikrodissektionssystem über ein Fluidsystem und/oder eine Elektrophoreseeinrichtung verfügen. Das Fluidsystem kann beispielsweise geeignete Pumpen umfassen, deren Aus- und/oder Eingänge mit den erläuterten Fluidleitungen verbunden werden können. Das Fluidsystem kann beispielsweise von einer Steuereinheit des Lasermikrodissektionssystems angesteuert werden. Entsprechendes gilt für eine Elektrophoreseeinrichtung, also eine Spannungsquelle, deren Aus- und/oder Eingänge mit den Anschlüssen der Elektroden der Elektrophoreseeinheit gekoppelt werden können. Die Erfindung schafft in dieser besonders vorteilhaften Ausgestaltung ein Lasermikrodissektionssystem mit einem vollintegrierten Elektrophoresesystem.

In bestimmten Fällen können jedoch auch Lasermikrodissektionssysteme vorteilhaft sein, bei denen die Elektrophoreseeinheit zur Durchführung eines Elektrophoreseverfahrens aus dem Lasermikrodissektionssystem entnehmbar ist.

Diese ist hierzu, wie oben erläutert, mit entsprechenden Kopplungsmitteln ausgebildet. Dies ermöglicht beispielsweise eine stärkere Modularisierung und die zentrale Auswertung von Dissektaten bzw. Elektrophoreseeinheiten unterschiedlicher Lasermikrodissektionssysteme an zentraler Stelle und unter identischen Bedingungen, beispielsweise zur Erhöhung der Reproduzierbarkeit. Ein entsprechender Gelträger kann in diesem Fall auch beispielsweise in geeigneter Weise konserviert und an ein Zentrallabor verschickt werden.

Besonders vorteilhaft ist ein Lasermikrodissektionssystem, das Auswertemittel zum Auswerten eines Ergebnisses des Elektrophoreseverfahrens aufweist. Wie erläutert, erlaubt das erfindungsgemäße Lasermikrodissektionssystem eine eindeutige Zuordnung von Geltaschen des Elektrophoresegels zu Bereichen der Probe. Hierbei kann ein entsprechendes Lasermikrodissektionssystem auch beispielsweise dazu ausgebildet sein, bei einer Vorgabe einer individuellen Geltasche die zugeordnete Position in dem jeweils zugehörigen Bereich der Probe anzufahren, so dass der Benutzer unmittelbar erkennen kann, welcher Probenbereich zu einem entsprechenden Signal in dem Elektrophoreseverfahren geführt hat. Umgekehrt kann auch vorgesehen sein, bei Vorgabe eines bestimmten Probenbereichs, beispielsweise durch Anfahren mittels eines positionierbaren Probenhalters, die jeweils zugehörigen Geltaschen (bzw. die zugehörigen Signale des Elektrophoreseverfahrens) anzuzeigen. Der Benutzer kann hierbei auch die Möglichkeit erhalten, eine Region der Probe auszuwählen (z.B. durch Einkreisen mittels einer digitalen Auswahleinheit). Das Lasermikrodissektionssystem zeigt auf dieser Grundlage alle in diesem Bereich erhaltenen Signale aus dem Elektrophoreseverfahren. Der Benutzer erhält hierdurch beispielsweise Informationen bezüglich einer Streuung der erhaltenen Signale in einem morphologisch einheitlichen Bereich der Probe. Entsprechende Auswertemittel umfassen daher beispielsweise Steuermittel, visuelle Anzeigemittel, Motoren zur Bewegung des Probenhalters und Auswahlmittel.

Insgesamt ergeben sich wesentliche Vorteile des erfindungsgemäßen Lasermikrodissektionssystems, wie erwähnt, daraus, dass jeweils eine der einen oder mehreren Geltaschen jeweils einer Region der nukleinsäurehaltigen Probe zuordenbar ist. Details wurden bereits erläutert.

Ein Elektrophoresegel für den Einsatz in der Erfindung kann beispielsweise 12, 24 oder 96 Geltaschen aufweisen. Diese sind beispielsweise in Form eines regelmäßigen Musters entsprechend bekannten Mikrotiterplatten (also in rechtwinklig zueinander angeordneten "Zeilen" und "Spalten") angeordnet. Die Anzahl der Geltaschen bestimmt deren Größe und räumliche Trennung. Die Anzahl der Geltaschen richtet sich damit auch nach der Probe und der Größe und/oder Anzahl der hieraus gewonnenen Dissektate. Wird viel Dissektatmaterial benötigt, oder sollen große Dissektate gewonnen werden, bietet sich die Verwendung einer geringeren Anzahl entsprechend größerer Geltaschen an. Dies gilt auch dann, wenn eine vergleichsweise lange Trennstrecke erwünscht ist. In diesem Fall muss ausreichend Gelmaterial zwischen den Geltaschen vorliegen, um diese Trennstrecke bereitzustellen. Zur Zugabe von Reagenzien kann beispielsweise auch eine unten offene Mikrotiterplatte verwendet werden, die auf ein entsprechendes Elektrophoresegel aufgesetzt wird (wie in dem erwähnten Artikel von Wood et al. beschrieben). In diesem Fall richtet sich die Anzahl und Anordnung der Geltaschen nach der Ausbildung einer derartigen Mikrotiterplatte.

Ein Elektrophoresegel kann vorgefertigt oder in einer entsprechenden Elektrophoreseeinheit durch ein bekanntes Gießverfahren hergestellt werden. Es kann auch vorgesehen sein, entsprechende Elektrophoreseeinheiten mit vorgefertigten Geleinsätzen zu versehen, was den Aufwand bei der Durchführung entsprechender Verfahren verringert. Die Geltaschen können beispielsweise durch die Verwendung geeigneter Matrizen beim Gießen des Elektrophoresegels in dieses eingebracht werden. Nach dem "Aushärten" des Gels kann die Matrize entnommen werden und das Gel liegt mit einer entsprechenden Anzahl an Geltaschen vor. Zur Einbringung der Geltaschen kann beispielsweise eine Matrize verwendet werden, die mittels gängiger Mikrostrukturtechniken erzeugt wurde.

Der Durchmesser bzw. das Volumen der Geltaschen des Elektrophoresegels richtet sich ferner auch nach der erwünschten Signalqualität, die, beispielsweise mittels eines Einzelzell-Gelektrophoreseverfahrens, erhalten werden soll. Typische Durchmesser bewegen sich in einem Bereich von 10 µm bis 50 µm. Größere Volumina ergeben stärkere, aber ggf. diffusere Signale. Bei geringeren Volumina werden schärfere, aber ggf. entsprechend schwächere Signale erhalten. Wie erwähnt, ist ein wesentlicher Faktor, der die Größe bzw. das Volumen der Geltaschen beeinflusst, die Menge der aufzunehmenden Probe.

Eine entsprechende Elektrophoreseeinheit für ein zuvor erläutertes Lasermikrodissektionssystem ist ebenfalls beschrieben.

Besonders vorteilhaft ist eine derartige Elektrophoreseeinheit, wenn sie Mittel zur reversiblen Anbringung in dem Lasermikrodissektionssystem aufweist. Diese können beispielsweise die oben erläuterten Anbringungsstrukturen umfassen. Hierbei kann, wie erwähnt, beispielsweise eine Entnahme zur Durchführung der Elektrophorese und/oder zur Einbringung eines Elektrophoresegels vorgenommen werden. Eine entsprechend modularisierte Ausbildung erlaubt die Vorbereitung bzw. Nachbearbeitung einer Elektrophoreseeinheit, während eine andere Elektrophoreseeinheit der Dissektion unterworfen oder ausgewertet wird.

In einem erfindungsgemäßen Untersuchungsverfahren für nukleinsäurehaltige Proben wird ein Lasermikrodissektionssystem wie zuvor erläutert verwendet. Das Verfahren umfasst, mittels eines Laserstrahls Dissektate aus einer nukleinsäurehaltigen Probe herauszutrennen, diese in einer oder mehreren Geltaschen des Elektrophoresegels aufzufangen, und diese anschließend mittels eines Elektrophoreseverfahrens zu untersuchen. Die Untersuchung selbst (d.h. das Elektrophoreseverfahren) kann in bekannter Weise (z.B. mittels des erwähnten Comet-Assays) erfolgen und eine Einbringung zusätzlichen Gelmaterials zur Abdeckung der Geltaschen und/oder von Reagenzien, Puffern, Enzymen, Fluoreszenzmarkern und dergleichen umfassen.

Wie mehrfach erwähnt, ist ein erfindungsgemäßes Untersuchungsverfahren dann besonders vorteilhaft, wenn jeweils eine zum Auffangen verwendete Geltasche der einen oder mehreren Geltaschen einer Region der Probe zugeordnet wird. Dies ermöglicht lokal aufgelöste Untersuchungen.

Wie ebenfalls erwähnt, können die Dissektate in einem "integrierten" Lasermikrodissektionssystem mittels des Elektrophoreseverfahrens untersucht werden, während die Elektrophoreseeinheit mit dem Elektrophoresegel in dem Lasermikrodissektionssystem angebracht ist. Vorteil kann hierbei eine zentrale Einstellmöglichkeit für sämtliche Parameter des Elektrophoreseverfahrens sein. Auf diese Weise kann beispielsweise ein vollständiges Bedienprogramm vorgegeben werden, in dem der Benutzer lediglich noch die zu dissektierenden Bereiche angeben muss. Das Lasermikrodissektionssystem führt in diesem Fall selbständig die weiteren Schritte aus und ist daher besonders benutzerfreundlich.

Zu den Vorteilen der alternativen Möglichkeit, die Dissektate mittels des Elektrophoreseverfahrens zu untersuchen, nachdem die Elektrophoreseeinheit mit dem Elektrophoresegel aus dem Lasermikrodissektionssystem entnommen wurde, sei auf die obigen Erläuterungen verwiesen.

Besonders vorteilhaft ist das Untersuchungsverfahren, wenn ferner ein Ergebnis des Elektrophoreseverfahrens mittels des Lasermikrodissektionssystems ausgewertet wird. Dies kann auch die mehrfach erwähnte Zuordnung umfassen.

Die vorliegende Erfindung und Ausführungsformen der Erfindung werden nachfolgend unter Bezugnahme auf die beigefügten Zeichnungen erläutert.

### Kurze Beschreibung der Zeichnungen

Figur 1 zeigt ein Lasermikrodissektionssystem, das zur Durchführung eines erfindungsgemäßen Verfahrens verwendet werden kann.
Figur 2 zeigt Details eines Lasermikrodissektionssystems gemäß Figur 1 in einer ersten Ausführungsform in schematischer Darstellung.
Figur 3 zeigt Details eines Lasermikrodissektionssystems gemäß Figur 1 in einer zweiten Ausführungsform in schematischer Darstellung.

In den Figuren werden einander entsprechende Elemente mit identischen Bezugszeichen angegeben und nicht wiederholt erläutert.

### Ausführungsform(en) der Erfindung

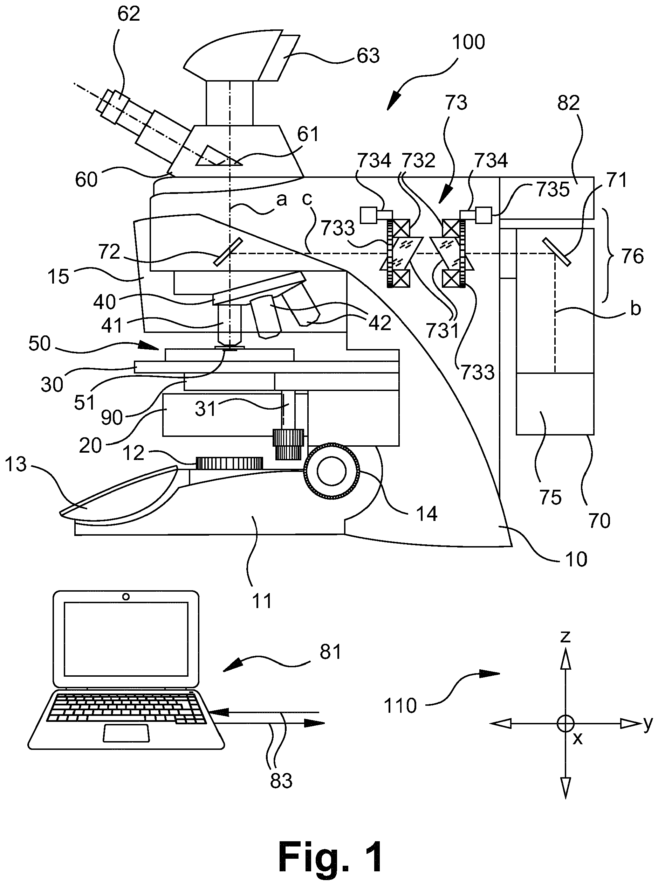

In Figur 1 ist ein Lasermikrodissektionssystem, das zur Durchführung eines erfindungsgemäßen Verfahrens verwendet werden kann, schematisch dargestellt und insgesamt mit 100 bezeichnet. Das Lasermikrodissektionssystem 100 entspricht in wesentlichen Teilen jenem, das in der EP 1 276 586 B1 offenbart ist, auf die hier ausdrücklich Bezug genommen wird. Ein Koordinatensystem, anhand dessen die nachfolgend erwähnten Achsen bzw. Richtungen x, y und z veranschaulicht sind, ist mit 110 bezeichnet.

Das Lasermikrodissektionssystem 100 umfasst ein Mikroskop 10. In einem Mikroskopfuß 11 des Mikroskops 10 kann eine hier nur teilweise dargestellte Beleuchtungseinrichtung 12 vorgesehen sein. Diese kann beispielsweise eine (nicht dargestellte) Lichtquelle und geeignete Mittel zur Beeinflussung des durch die Lichtquelle bereitgestellten Beleuchtungslichts umfassen, beispielsweise Filter und/oder Blenden.

Am Mikroskopfuß 11 kann beispielsweise auch eine Benutzereingabe- und/oder Benutzerinformationseinheit 13 angeordnet sein, die beispielsweise als Touchscreen ausgebildet sein kann, und über die der Benutzer beispielsweise Betrachtungs- und/oder Bearbeitungsparameter eingeben und/oder auslesen kann.

Ferner ist ein Triebknopf 14 vorgesehen. Dieser dient zur Bedienung eines Grob- und eines Feintriebs zur Einstellung einer Höhe eines Mikroskoptischs 30. Eine Probe 51, die sich auf einem Objektträger (hier ohne Bezeichnung) auf einem Probenhalter 50 befindet, beispielsweise ein auf dem Objektträger aufgebrachter Schnitt, kann hierdurch in eine Schärfenebene eines Objektivs 41 gebracht werden. Das Objektiv 41 ist neben weiteren Objektiven 42 in einem Objektivrevolver 40 befestigt. Zum Schutz vor Laserstrahlung kann eine Schutzhaube 15 vorgesehen sein. Zur Durchlichtbeleuchtung der Probe 51 und zur Einstellung geeigneter Kontrast- bzw. Beobachtungsverfahren dient eine Kondensoreinheit 20.

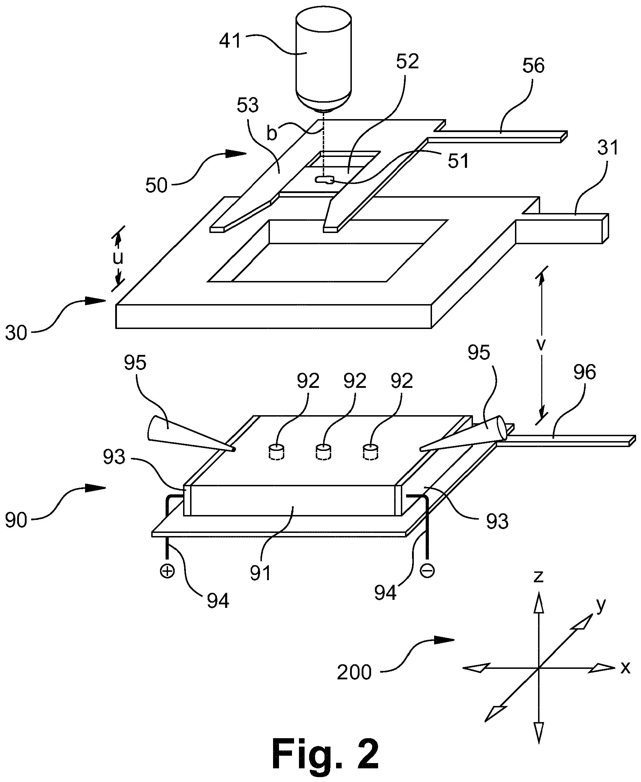

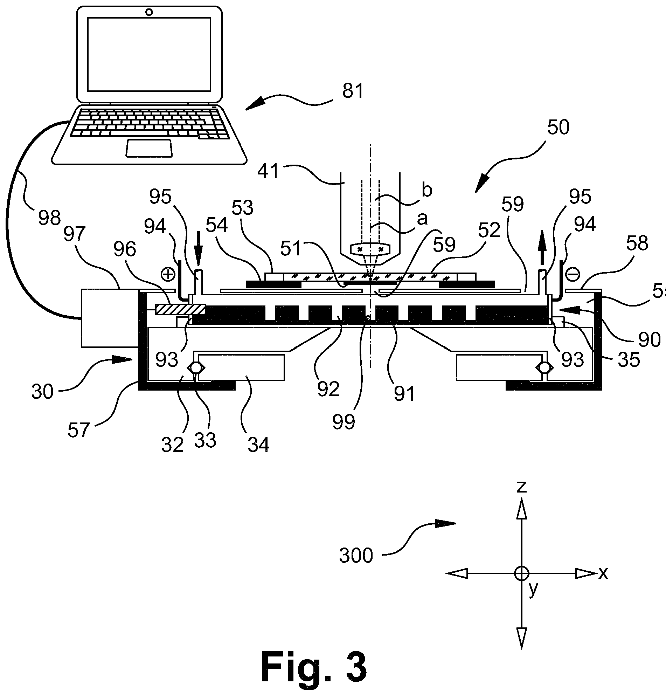

Unterhalb des Probenhalters 50 befindet sich eine Elektrophoreseeinheit 90, die mit einem Elektrophoresegel mit geeigneten Geltaschen ausgestattet werden kann. Details hierzu sind in den nachfolgenden Figuren 2 und 3 gezeigt.

Von der Probe 51 ausgehendes Beobachtungslicht verläuft entlang eines Beobachtungsstrahlengangs a. In einer Tubuseinheit 60 mit geeigneten Auskoppeleinrichtungen 61 kann ein vorzugsweise variabler Anteil des Beobachtungslichts, beispielsweise um 60°, ausgekoppelt und mittels eines Okularpaars 62 einem Benutzer dargeboten werden. Ein weiterer Anteil des Beobachtungslichts kann in eine digitale Bilderfassungseinheit 63 eingekoppelt und bildgebend erfasst werden.

Das Lasermikrodissektionssystem 100 weist eine Lasereinheit 70 mit einer Laserlichtquelle 75 auf. Ein durch die Laserlichtquelle 75, bei der es sich beispielsweise um eine UV-Laserlichtquelle handeln kann, bereitgestellter Laserstrahl b wird in einer Auflichteinheit, die hier insgesamt mit 76 angegeben ist, an einem ersten Umlenkspiegel 71 und einem zweiten Umlenkspiegel 72 umgelenkt und durch das Objektiv 41 auf die Probe 51 fokussiert.

Bei dem Lasermikrodissektionssystem 100 kann der Ort, an dem der Laserstrahl b auf die Probe 51 auftrifft, grundsätzlich auf unterschiedliche Weise eingestellt werden. Einerseits kann eine manuelle Verstelleinrichtung 31 vorgesehen sein, mittels derer der als Kreuztisch ausgebildete Mikroskoptisch 30 in x- und y-Richtung (also hier senkrecht bzw. parallel zur Papierebene) verstellt werden kann. Neben der Verstelleinrichtung 31 können auch elektromechanische Stellmittel vorgesehen sein, die beispielsweise durch eine Steuereinheit 82 angesteuert bzw. deren Position durch die Steuereinheit 82 erfasst werden kann.

Die Steuereinheit 82 kann auch beliebige weitere motorisierter Funktionen des Lasermikrodissektionssystems 100 steuern und insbesondere eine Schnittstelle zu einem externen Steuerrechner 81, der über entsprechende Verbindungen 83 angebunden sein kann, bereitstellen. Insbesondere sind der Steuerrechner 81 und/oder die Steuereinheit 82 als Mittel zur Ansteuerung des Objekttischs 30, des Probenhalters 50 und/oder der Elektrophoreseeinheit 90 (siehe Figuren 2 und 3) eingerichtet.

Für die Lasermikrodissektion kann jedoch insbesondere eine Laserscaneinrichtung 73 vorgesehen sein. Mittels der Laserscaneinrichtung 73 kann der Laserstrahl b auch gegenüber einer zwischen dem ersten Umlenkspiegel 71 und dem zweiten Umlenkspiegel 72 verlaufenden optischen Achse c abgelenkt werden. Der Laserstrahl kann daher an unterschiedlichen Positionen auf den zweiten Umlenkspiegel 72 auftreffen, der beispielsweise als dichromatischer Teiler ausgebildet sein kann, und wird damit auch an unterschiedlichen Positionen auf die Probe 51 fokussiert. Eine entsprechende Ablenkung mittels einer Laserscaneinrichtung 73 ist im Detail in der EP 1 276 586 B1 gezeigt. Es sei betont, dass unterschiedliche Möglichkeiten zur Ablenkung eines Laserstrahls b bzw. zur Positionierung der Probe 51 gegenüber dem Laserstrahl b zum Einsatz kommen können. Die Erfindung ist nicht auf das dargestellte Beispiel beschränkt.

Im dargestellten Beispiel weist die Laserscaneinrichtung 73 zwei massive gläserne Keilplatten 731 auf, die gegen die optische Achse c geneigt und unabhängig voneinander um die optische Achse c drehbar sind. Hierzu sind die Keilplatten 731 mit Kugellagern 732 gelagert. Jede der Keilplatten ist mit einem Zahnrad 733 verbunden. Die Zahnräder 733 können jeweils mittels Rotationseinrichtungen 734 gedreht werden. Die Rotationseinrichtungen 734 können manuell und/oder mittels geeigneter elektromechanischer Vorrichtungen, beispielsweise mittels Schrittmotoren, in eine Drehbewegung versetzt werden und hierüber die Zahnräder 733 antreiben. Die Rotationseinrichtungen 734 können über Positonsgeber 735 verfügen (hier nur an der rechten Rotationseinrichtung 734 gezeigt). Eine hierdurch erfasste Position kann an die Steuereinheit 80 übermittelt werden.

Figur 2 zeigt Details eines Lasermikrodissektionssystems 100 gemäß Figur 1 in einer ersten Ausführungsform in schematischer Darstellung. Hierbei sind jeweils Teile des Probenhalters 50, des Mikroskoptischs 30 und der Elektrophoreseeinheit 90 dargestellt. Ferner ist das Mikroskopobjektiv 41 gezeigt. Die genannten Elemente sind perspektivisch und stark vereinfacht gezeigt. Das verwendete Koordinatensystem ist mit 200 bezeichnet. Die Richtungsangaben x, y und z entsprechen jenen der Figur 1. Die Achsen bzw. Richtungen x und z liegen in der Papierebene.

Der Probenhalter 50 umfasst eine Halteeinrichtung 53, mittels derer beispielsweise ein Objektträger 52 mit der Probe 51 gehaltert werden kann. Die Halteeinrichtung 53 kann über eine Verschiebeeinrichtung 56 in zumindest einer der Richtungen x und y verschoben werden. Hierdurch ist es möglich, Bereiche der Probe 51 gegenüber dem Laserstrahl b zu verschieben. Die Verschiebeeinrichtung 56 ist stark schematisiert dargestellt. Ein erfindungsgemäßes Lasermikrodissektionssystem 100 kann beispielsweise koaxial angeordnete Rändelschrauben bzw. Drehknöpfe umfassen, mittels derer, wie für die Verstelleinrichtung 31 des Objekttischs 30 in Figur 1 gezeigt, die Halteeinrichtung 53, und damit Die Probe 51 in x- und y-Richtung verschoben werden kann.

Wie nachfolgend unter Bezugnahme auf die Figur 3 veranschaulicht, befindet sich die Probe 51 üblicherweise auf der Unterseite eines Objektträgers 52. Wenngleich der Objektträger 52 im dargestellten Beispiel quadratisch ausgebildet ist, kann ein erfindungsgemäßes Lasermikrodissektionssystem auch mit einer Halteeinrichtung 53 ausgestattet sein, die zur Halterung herkömmlicher Objektträger im Seitenverhältnis von 1 : 3 oder 1 : 4 ausgebildet ist.

Der Probenhalter 50 ist oberhalb (in z-Richtung, d.h. betrachter- bzw. objektivseitig) des Mikroskoptischs 30 angeordnet. Auch der Mikroskoptisch 30 kann, insbesondere mittels der hier stark schematisiert dargestellten Verstelleinrichtung 31, zumindest in einer der Richtungen x und y bewegt werden. Auch hier können beispielsweise koaxiale Rändelschrauben vorgesehen sein. Wenngleich im dargestellten Beispiel ein Abstand u zwischen dem Probenhalter 50 und dem Probentisch 30 gezeigt ist, kann der Probenhalter 50 auch direkt auf dem Probentisch 30 angeordnet sein. Insbesondere kann der Abstand u auch variabel ausgebildet sein.

Im dargestellten Beispiel ist beleuchtungsseitig, also in z-Richtung unterhalb des Objekttischs 30 die Elektrophoreseeinheit 90 angeordnet. Auch die Elektrophoreseeinheit 90 kann durch geeignete Verstellmittel 96 verstellbar ausgebildet sein. Auch hier kann eine Verstellung in zumindest einer der Richtungen x und y erfolgen. Die Elektrophoreseeinheit 90 ist in der Figur 2 stark vereinfacht dargestellt. Auch hier kann ein Abstand v zwischen der Elektrophoreseeinheit 90 und dem Probentisch 30 abweichend zu der Darstellung der Figur 2 ausgebildet sein. Insbesondere kann die Elektrophoreseeinheit 90 direkt unterhalb des Objekttischs 30 angeordnet sein.

Die Elektrophoreseeinheit 90 weist ein Elektrophoresegel 91 auf, das im dargestellten Beispiel drei Geltaschen 92 aufweist. Die Geltaschen 92 können in jeder beliebigen Anzahl, Form und Größe in dem Elektrophoresegel ausgebildet sein. Die Geltaschen 92 können beispielsweise unter Verwendung einer geeigneten Matrize erzeugt werden, die in ein geschmolzenes Gelmaterial, das zur Erzeugung des Elektrophoresegels 91 verwendet wird, beispielsweise in geschmolzene Agarose, gedrückt wird. Nach dem Aushärten des Gelmaterials liegen die Geltaschen 92 in der gewünschten Ausformung vor. Bei den hier dargestellten Elektrophoresegel 91 werden beispielsweise 24, 96 oder 384 Geltaschen verwendet. Die einzelnen Geltaschen können Dimensionen von deutlich unter 100 µm aufweisen. Üblich sind beispielsweise Durchmesser von 10 µm bis 50 µm.

Das Elektrophoresegel 91 ist zwischen Elektroden 93 angeordnet, an die über Anschlüsse 94 eine Spannung (mit + und - veranschaulicht) angelegt werden kann. Die Spannung kann, beispielsweise mittels der Steuereinheit 82 und/oder des Steuerrechners 81, eingestellt werden. Eine geeignete Spannungsquelle ist vorgesehen, der Übersichtlichkeit halber jedoch nicht dargestellt.

Wie allgemein bekannt, erfolgt die Trennung von Proben in einem Elektrophoresegel unter Verwendung eines geeigneten Trennpuffers. Dieser kann über ein Fluidsystem mit entsprechenden Fluidleitungen 95 in die Elektrophoreseeinheit 90 eingebracht und aus dieser abgezogen werden. Mittels der Fluidleitungen 95 können beispielsweise auch geeignetes Färbereagenz, radioaktive Labels und/oder geeignete Enzyme in die Elektrophoreseeinheit 90 eingebracht werden. Auch die Einbringung entsprechender Fluide bzw. deren Entnahme über die jeweiligen Fluidleitungen 95 kann mittels des Steuerrechners 81 und/oder der Steuereinheit 82 gesteuert werden. Hierzu sind vorteilhafterweise geeignete Pumpen vorgesehen, die jedoch der Übersichtlichkeit halber ebenfalls nicht veranschaulicht sind. Wie erwähnt, kann auch vorgesehen sein, die Elektrophoreseeinheit 90 aus dem Lasermikrodissektionssystem 100 zu entnehmen.

In Figur 3 ist ein Querschnitt durch den Mikroskoptisch 30 mit dem darauf angebrachten Probenhalter 50 in einer durch die optische Achse des Beobachtungsstrahlengangs a und die x-Richtung (senkrecht zur Papierebene der Figur 1) aufgespannten Ebene dargestellt. Das verwendete Koordinatensystem mit den Richtungen bzw. Achsen ist mit 300 bezeichnet.

Der Mikroskoptisch 30 weist eine feststehende Basisplatte 34 auf, auf der eine in y-Richtung (also senkrecht zur Papierebene) verfahrbare Platte 32 angeordnet ist. Die verfahrbare Platte 32 definiert eine Tischoberfläche (ohne Bezeichnung) des Mikroskoptischs 30. Die verfahrbare Platte 32 ist gegenüber der feststehenden Basisplatte 34 mittels einer geeigneten Einrichtung, beispielsweise unter Verwendung von Lagerkugeln 33, verschiebbar. Zum wechselseitigen Verschieben der feststehenden Basisplatte 34 und der verfahrbaren Platte 32 kann die bereits erläuterte Verstelleinrichtung 31 verwendet werden. Ist hier davon die Rede, dass die feststehende Basisplatte 34 "feststeht", bezieht sich dies nur auf ihre Position gegenüber der verfahrbaren Platte 32. Die "feststehende" Basisplatte 34 kann auch ihrerseits - in x-Richtung - gegenüber einer weiteren Platte des Mikroskoptischs 30 verschoben und - in z-Richtung - in ihrer Höhe verstellt werden.

An der feststehenden Basisplatte 34 sind zwei Halteelemente 57 vorgesehen, von denen nur das linke Halteelement 57 mit einem Bezugszeichen versehen ist. Die Halteelemente 57 tragen eine Kontaminationsschutzplatte 58, die zwischen dem Trägerelement 54 (s.u.) und der Elektrophoreseeinheit 90 feststeht. Die Kontaminationsschutzplatte 58 überspannt vorteilhafterweise die gesamte Tischoberfläche des Mikroskoptischs 30 und begrenzt damit einen freien Arbeitsraum 55 nach oben. Die Kontaminationsschutzplatte 58 ist mit Ausschnitten 59 versehen.

Der Probenhalter 50 weist ein Trägerelement 54 auf, auf das ein Objektträger 52 aufgelegt werden kann. Die Probe 51 ist an der Unterseite des Objektträgers 52 angebracht. Der Objektträger 52 mit der Probe 51 kann mittels der zuvor unter Bezugnahme auf Figur 2 erläuterten Halteeinrichtung 53 in x- und/oder y-Richtung verschoben werden.

Auf der Tischoberfläche des Mikroskoptischs 30 ist die Elektrophoreseeinheit 90 angeordnet. Die Anordnung im Beispiel der Figur 3 unterscheidet sich damit von jener der Figur 2 dadurch, dass die Elektrophoreseeinheit 90 in der Figur 2 unterhalb, in der Figur 3 hingegen oberhalb des Objekttischs 30 angeordnet ist.

Die Elektrophoreseeinheit 90 ist in den freien Arbeitsraum 55 eingebracht und kann beispielsweise mittels Kopplungsmitteln 35 in dem

Lasermikrodissektionssystem 100 angebracht werden. Die Elektrophoreseeinheit 90 weist die grundlegenden Elemente auf, die bereits unter Bezugnahme auf Figur 2 erläutert wurden. Hierbei handelt es sich insbesondere um ein Elektrophoresegel 91, hier schraffiert dargestellt, in das die Geltaschen 92 eingebracht sind. Die Elektroden 93 sind über entsprechende Leitungen 94 angeschlossen, die Fluidleitungen 95 können zum Einbringen bzw. Ableiten geeigneter Fluide verwendet werden, wie hier durch Pfeile veranschaulicht. Die Elektrophoreseeinheit 90 kann auch in einer geeigneten Kammer eingeschlossen sein, wobei sichergestellt wird, dass Dissektate der Probe 51 in die jeweiligen Geltaschen 92 fallen können. Für die Leitungen 94 bzw. 95 können ebenfalls geeignete Aussparungen 59 der Kontaminationsschutzplatte 58 vorgesehen sein. Auf die Verwendung einer Kontaminationsschutzplatte 58 kann auch verzichtet werden.

Das Mikroskopobjektiv 51 ist auf der optischen Achse des Beobachtungsstrahlengangs a (hier wie in Figur 1 strichpunktiert dargestellt) dem Objektträger 52 zugewandt und erzeugt über eine entsprechende Linsenanordnung (ohne Bezeichnung) ein Bild der Probe 51. Die Probe 51 befindet sich hierzu in einer Brennebene des Mikroskopobjektivs 41. Ferner wird durch das Mikroskopobjektiv 41 ein wie zu Figur 1 erläutert eingekoppelter Laserstrahl b auf die Probe 51 fokussiert, um aus der Probe 51 ein entsprechendes Dissektat, beispielsweise eine oder mehrere Einzelzellen, auszuschneiden.

Jeweils eine Geltasche 92 des Elektrophoresegels 91 ist derart unter der Probe 51 angeordnet, dass das Dissektat aufgrund der Schwerkraft in dieses herabfällt. Ein Dissektat in einer Geltasche 92 ist stark schematisiert dargestellt und mit 99 bezeichnet. Die Anzahl der Geltaschen 92 kann, wie erläutert, an die jeweiligen Erfordernisse, beispielsweise die Anzahl der zu gewinnenden Dissektate, angepasst werden. Ist eine Kontaminationsschutzplatte 58 vorgesehen, kann jeweils eine Geltasche 92 des Elektrophoresegels 91 unterhalb des Ausschnitts 59 auf der optischen Achse des Beobachtungsstrahlengangs a angeordnet werden. Durch die Kontaminationsschutzplatte 58 wird verhindert, dass Staub oder andere Partikel aus der Umgebungsluft in die jeweilige Geltasche 92 fallen. Ferner wird verhindert, dass Dissektate 99 der Probe 51 in eine "falsche" Geltasche 92 fallen, wenn sie aus der Probe 51 herausgeschleudert werden. Mit anderen Worten können, bis auf die gewünschte Geltasche 92, sämtliche anderen Geltaschen 92 durch die Kontaminationsschutzplatte 58 verschlossen werden. Dies ermöglicht eine effektive Analyse und die Vermeidung von Artefakten.

Mit der Verstelleinrichtung 96 können geeignete Stellmittel 97 verbunden werden. Die Stellmittel 97 können für eine manuelle Bedienung ausgebildet sein, weisen jedoch vorzugsweise einen entsprechenden Motor auf, mittels dessen die Verstelleinrichtung 96 und die daran angebrachte Elektrophoreseeinheit 90 verfahren werden kann. Die Stellmittel 97 sind hierzu über eine entsprechende Verbindung 98 mit einem Rechner, beispielsweise dem erläuterten Steuerrechner 81, verbunden. Der Steuerrechner 81 oder eine Steuereinheit 82 verschiebt über die Stellmittel 97 bzw. die Verstelleinrichtung 96 die Elektrophoreseeinheit 90 bzw. das Elektrophoresegel 91 derart, dass sich jeweils die gewünschte Geltasche 92 unter dem Ausschnitt 59 auf der optischen Achse des Beobachtungsstrahlengangs a und damit unter dem Bereich der Probe 51 befindet, der mittels des Laserstrahls b bearbeitet wird. Nachdem jeweils ein Lasermikrodissektionsvorgang abgeschlossen ist, fällt das entsprechende Dissektat 99 aufgrund der Schwerkraft in die jeweilige Geltasche 92. Der Steuerrechner 81 wird dann, falls erforderlich, eine andere oder leere Geltasche 92 entsprechend positionieren, um damit ein oder mehrere neue Dissektate aufzufangen.

Nachdem eine gewünschte Anzahl an Dissektaten gewonnen und in den Geltaschen 92 aufgenommen wurde, kann eine Elektrophorese eingeleitet werden, indem beispielsweise ein geeigneter Puffer über die Fluidleitungen 95 in die Elektrophoreseeinheit 91 eingebracht wird. Zuvor kann das Elektrophoresegel 91 auch mit Gelmaterial, das über die Fluidleitungen 95 zugeführt wird, überschichtet werden. Durch Anlegen einer Spannung über die Leitungen 94 an die Elektroden 93 wird ein Spannungsgradient erzeugt, der eine Elektrophorese der jeweils aufgenommenen Dissektate bewirkt. Insbesondere kann in diesem Zusammenhang der eingangs erläuterte sogenannte Comet-Assay durchgeführt werden.

Nach der Durchführung des Elektrophoreseverfahrens kann dessen Erfolg direkt über das Objektiv 41 überprüft werden.

## Patentansprüche

1. Lasermikrodissektionssystem (100) mit einem Mikroskop (10), das eine Auflichteinrichtung (76), ein Mikroskopobjektiv (41) und eine Lasereinheit (70) aufweist, wobei ein Strahlengang (b) eines Laserstrahls der Lasereinheit (70) durch die Auflichteinrichtung (76) und durch das Mikroskopobjektiv (41) verläuft und eine Objektebene (15) des Mikroskopobjektivs (41) an einem einstellbaren Schnittpunkt schneidet, **dadurch gekennzeichnet, dass** unterhalb der Objektebene (15) eine Elektrophoreseeinheit (90) mit einem Elektrophoresegel (91) angebracht ist, wobei das Elektrophoresegel (91) zumindest eine Geltasche (92) aufweist und das Lasermikrodissektionssystem (100) Positionierungsmittel (96) zum Positionieren des Elektrophoresegels (91) parallel zu der Objektebene (15) und relativ zu einer definierten Bezugsposition umfasst, so dass mittels des Laserstrahls der Lasereinheit (70) gewonnene Dissektate (99) einer in der Objektebene (15) anordenbaren Probe (51) in der zumindest einen Geltasche (92) auffangbar sind, wobei die Positionierungsmittel (96) geeignet sind, um das Elektrophoresegel (91) relativ zu der Probe (51) zu positionieren.

2. Lasermikrodissektionssystem (100) nach Anspruch 1, bei dem die Elektrophoreseeinheit (90) Fluidleitungen (95) zum Einbringen von Fluid in die Elektrophoreseeinheit (90) und/oder zur Entnahme von Fluid aus dieser aufweist.

3. Lasermikrodissektionssystem (100) nach Anspruch 1 oder 2, bei dem die Elektrophoreseeinheit (90) Elektroden (93) zum Anlegen einer Spannung an das Elektrophoresegel (91) aufweist.

4. Lasermikrodissektionssystem (100) nach einem der vorstehenden Ansprüche, das ein Fluidsystem und/oder eine Elektrophoreseeinrichtung zur Durchführung eines Elektrophoreseverfahrens mittels der in dem Lasermikrodissektionssystem (100) angebrachten Elektrophoreseeinheit (91) aufweist.

5. Lasermikrodissektionssystem (100) nach einem der Ansprüche 1 bis 3, bei dem die Elektrophoreseeinheit (90) zur Durchführung eines Elektrophoreseverfahrens aus dem Lasermikrodissektionssystem (100) entnehmbar ist.

6. Lasermikrodissektionssystem (100) nach einem der Ansprüche 4 oder 5, das Auswertemittel zum Auswerten eines Ergebnisses des durchgeführten Elektrophoreseverfahrens aufweist.

7. Lasermikrodissektionssystem (100) nach einem der vorstehenden Ansprüche, das Mittel zum Zuordnen einer oder mehrerer der einen oder mehreren Geltaschen (92) zu jeweils einer oder mehreren Regionen der Probe (51) aufweist.

8. Lasermikrodissektionssystem (100) nach einem der vorstehenden Ansprüche, bei dem das Elektrophoresegel (91) 12, 24 oder 96 Geltaschen (92) aufweist.

9. Lasermikrodissektionssystem (100) nach einem der vorstehenden Ansprüche, bei dem zumindest eine der einen oder mehreren Geltaschen (92) des Elektrophoresegels (91) einen Durchmesser von 10 µm bis 50 µm aufweist.

10. Untersuchungsverfahren für nukleinsäurehaltige Proben (51), bei dem ein Lasermikrodissektionssystem (100) nach einem der Ansprüche 1 bis 9 verwendet wird, wobei mittels eines Laserstrahls (b) Dissektate (99) aus einer nukleinsäurehaltigen Probe (51) herausgetrennt, in einer oder mehreren Geltaschen (92) des Elektrophoresegels (91) aufgefangen, und anschließend mittels eines Elektrophoreseverfahrens untersucht werden.

11. Untersuchungsverfahren nach Anspruch 10, bei dem jeweils eine zum Auffangen verwendete Geltasche (92) der einen oder mehreren Geltaschen (92) einer Region der Probe (51) zugeordnet wird.

12. Untersuchungsverfahren nach Anspruch 10 oder 11, bei dem die Dissektate mittels des Elektrophoreseverfahrens untersucht werden, während die Elektrophoreseeinheit (90) mit dem Elektrophoresegel (91) in dem Lasermikrodissektionssystem (100) angebracht ist.

13. Untersuchungsverfahren nach Anspruch 10 oder 11, bei dem die Dissektate mittels des Elektrophoreseverfahrens untersucht werden, nachdem die Elektrophoreseeinheit (90) mit dem Elektrophoresegel (91) aus dem Lasermikrodissektionssystem (100) entnommen wurde.

14. Untersuchungsverfahren nach einem der Ansprüche 10 bis 13, bei dem ferner ein Ergebnis des Elektrophoreseverfahrens mittels des Lasermikrodissektionssystems (100) ausgewertet wird.

## Claims

1. Laser microdissection system (100) comprising a microscope (10) which has a reflected-light device (76), a microscope objective (41) and a laser unit (70), wherein the beam path (b) of a laser beam of the laser unit (70) runs through the reflected-light device (76) and through the microscope objective (41) and intersects an object plane (15) of the microscope objective (41) at an adjustable point of intersection, **characterized in that** an electrophoresis unit (90) with an electrophoresis gel (91) is attached below the object plane (15), wherein the electrophoresis gel (91) has at least one gel pocket (92) and the laser microdissection system (100) comprises positioning means (96) for positioning the electrophoresis gel (91) parallel to the object plane (15) and relative to a defined reference position such that dissectates (99) which are of a specimen (51) that is arrangeable in the object plane (15) and which are obtained by means of the laser beam of the laser unit (70) are able to be captured in the at least one gel pocket (92), wherein the positioning means (96) are suitable for positioning the electrophoresis gel (91) relative to the specimen (51).

2. Laser microdissection system (100) according to Claim 1, wherein the electrophoresis unit (90) has fluid lines (95) for introducing fluid into the electrophoresis unit (90) and/or for removing fluid from same.

3. Laser microdissection system (100) according to Claim 1 or 2, wherein the electrophoresis unit (90) has electrodes (93) for applying a voltage to the electrophoresis gel (91).

4. Laser microdissection system (100) according to any one of the preceding claims, having a fluid system and/or an electrophoresis device for carrying out an electrophoresis method by means of the electrophoresis unit (91) attached in the laser microdissection system (100) .

5. Laser microdissection system (100) according to any one of Claims 1 to 3, wherein the electrophoresis unit (90) is removable from the laser microdissection system (100) for the purposes of carrying out an electrophoresis method.

6. Laser microdissection system (100) according to either of Claims 4 and 5, having evaluation means for evaluating a result of the performed electrophoresis method.

7. Laser microdissection system (100) according to any one of the preceding claims, having means for assigning one or more of the one or more gel pockets (92) to one or more regions of the specimen (51) in each case.

8. Laser microdissection system (100) according to any one of the preceding claims, wherein the electrophoresis gel (91) has 12, 24 or 96 gel pockets (92).

9. Laser microdissection system (100) according to any one of the preceding claims, wherein at least one of the one or more gel pockets (92) of the electrophoresis gel (91) has a diameter ranging from 10 µm to 50 µm.

10. Examination method for nucleic acid-containing samples (51), wherein use is made of a laser microdissection system (100) according to any one of Claims 1 to 9, wherein dissectates (99) are separated from a nucleic acid-containing specimen (51) by means of a laser beam (b), are captured in one or more gel pockets (92) of the electrophoresis gel (91) and are subsequently examined by means of an electrophoresis method.

11. Examination method according to Claim 10, wherein one gel pocket (92) of the one or more gel pockets (92) used for capturing purposes is respectively assigned to one region of the specimen (51).

12. Examination method according to Claim 10 or 11, wherein the dissectates are examined by means of the electrophoresis method while the electrophoresis unit (90) with the electrophoresis gel (91) is attached in the laser microdissection system (100).

13. Examination method according to Claim 10 or 11, wherein the dissectates are examined by means of the electrophoresis method after the electrophoresis unit (90) with the electrophoresis gel (91) was removed from the laser microdissection system (100).

14. Examination method according to any one of Claims 10 to 13, wherein further a result of the electrophoresis method is evaluated by means of the laser microdissection system (100).

## Revendications

1. Système de microdissection laser (100) muni d'un microscope (10) qui comporte un dispositif à lumière incidente (76), un objectif de microscope (41) et une unité laser (70), un trajet de faisceau (b) d'un faisceau laser de l'unité laser (70) passant à travers le dispositif à lumière incidente (76) et à travers l'objectif de microscope (41) et coupant un plan objet (15) de l'objectif de microscope (41) à une intersection réglable, **caractérisé en ce qu'**une unité d'électrophorèse (90) pourvue d'un gel d'électrophorèse (91) est montée au-dessous du plan objet (15),
le gel d'électrophorèse (91) comportant au moins une poche de gel (92) et le système de microdissection laser (100) comprenant des moyens de positionnement (96) destinés à positionner le gel d'électrophorèse (91) parallèlement au plan objet (15) et par rapport à une position de référence définie, de sorte que des matières disséquées (99), obtenues au moyen du faisceau laser de l'unité laser (70), d'un échantillon (51) qui peut être disposé dans le plan objet (15) peuvent être collectées dans l'au moins une poche de gel (92),
les moyens de positionnement (96) étant adaptés pour positionner le gel d'électrophorèse (91) par rapport à l'échantillon (51).

2. Système de microdissection laser (100) selon la revendication 1, dans lequel l'unité d'électrophorèse (90) comporte des conduites de fluide (95) destinées à introduire un fluide dans l'unité d'électrophorèse (90) et/ou à retirer un fluide de celle-ci.

3. Système de microdissection laser (100) selon la revendication 1 ou 2, dans lequel l'unité d'électrophorèse (90) comporte des électrodes (93) destinées à appliquer une tension au gel d'électrophorèse (91) .

4. Système de microdissection laser (100) selon l'une des revendications précédentes, lequel comporte un système fluidique et/ou un dispositif d'électrophorèse destinés à mettre en œuvre un procédé d'électrophorèse au moyen de l'unité d'électrophorèse (91) montée dans le système de microdissection laser (100).

5. Système de microdissection laser (100) selon l'une des revendications 1 à 3, dans lequel l'unité d'électrophorèse (90) peut être retirée du système de microdissection laser (100) pour mettre en œuvre un procédé d'électrophorèse.

6. Système de microdissection laser (100) selon l'une des revendications 4 ou 5, lequel comporte des moyens d'évaluation destinés à évaluer un résultat du processus d'électrophorèse mis en œuvre.

7. Système de microdissection laser (100) selon l'une des revendications précédentes, lequel comporte des moyens destinés à associer une ou plusieurs des une ou plusieurs poches de gel (92) respectivement à une ou plusieurs régions de l'échantillon (51).

8. Système de microdissection laser (100) selon l'une des revendications précédentes, dans lequel le gel d'électrophorèse (91) comporte 12, 24 ou 96 poches de gel (92) .

9. Système de microdissection laser (100) selon l'une des revendications précédentes, dans lequel au moins une des une ou plusieurs poches de gel (92) du gel d'électrophorèse (91) a un diamètre de 10 µm à 50 µm.

10. Procédé d'examen d'échantillons (51) contenant des acides nucléiques, dans lequel un système de microdissection laser (100) selon l'une des revendications 1 à 9 est utilisé, des matières disséquées (99) étant séparées d'un échantillon (51) contenant des acides nucléiques au moyen d'un faisceau laser (b), collectées dans une ou plusieurs poches de gel (92) du gel d'électrophorèse (91) puis examinées au moyen d'un procédé d'électrophorèse.

11. Procédé d'examen selon la revendication 10, dans lequel une poche de gel (92), utilisée pour la collecte, de l'une ou plusieurs poches de gel (92) est associée à une région de l'échantillon (51).

12. Procédé d'examen selon la revendication 10 ou 11, dans lequel les matières disséquées sont examinées au moyen du procédé d'électrophorèse tandis que l'unité d'électrophorèse (90) pourvue du gel d'électrophorèse (91) est fixée dans le système de microdissection laser (100) .

13. Procédé d'examen selon la revendication 10 ou 11, dans lequel les matières disséquées sont examinées au moyen du procédé d'électrophorèse après que l'unité d'électrophorèse (90) pourvue du gel d'électrophorèse (91) a été retirée du système de microdissection laser (100) .

14. Procédé d'examen selon l'une des revendications 10 à 13, dans lequel un résultat du procédé d'électrophorèse est en outre évalué au moyen du système de microdissection laser (100).
